# EUROPEAN PATENT SPECIFICATION

(11) **EP 0 717 613 B1**
(45) Date of publication and mention of the grant of the patent: **27.12.2000**
(21) Application number: 94925497.3
(22) Date of filing: 31.08.1994
(51) Int. Cl.: A61K 7/48, A61K 35/78

(54) **STABLE COMPOSITION CONTAINING WHEAT SPROUT JUICE AND PROCESS FOR PRODUCING IT**
WEIZENSPROSSENSAFT ENTHALTENDES STABILES PRÄPARAT UND VERFAHREN ZU SEINER HERSTELLUNG
COMPOSITION STABLE CONTENANT DU JUS DE GERME DE BLE ET PROCEDE DE PRODUCTION

(30) Priority: 01.09.1993 FI 933833
(43) Date of publication of application: 26.06.1996
(73) Proprietor: HELSINKI UNIVERSITY LICENSING LTD. OY, 00710 Helsinki (FI)
(72) Inventor: HILTUNEN, Raimo, FIN-00180 Helsinki (FI); VUORELA, Heikki, FIN-06880 Kärrby (FI)
(74) Representative: Lax, Monica Ingeborg
(86) International application number: FI9400384
(87) International publication number: WO9506459

(56) References cited:
- EP-A- 0 279 984
- WO-A-85/00746
- FR-A- 2 663 848

## Description

The invention relates to a composition containing wheat sprout juice and thyme oil. The composition is suitable for both cosmetic and pharmaceutical uses, and it is preferably formulated as a cream preparation. The invention further relates to a process for producing the composition. The invention also relates to a process for stabilizing wheat sprout juice or a composition containing it by thyme oil.

Wheat sprout is extremely complex in composition. The crude protein content of wheat sprout is high. Amino acid analyses have shown that especially the amounts of aspartic acid and essential amino acids, i.e., lysin, threonine, valine, leucine, isoleucine, methionine, phenylalanine and tryptophan, are high. Wheat sprout also contains high concentrations of arginine, proline, serine, tyrosine and cystine, which amino acids have a growth promoting effect. The chlorophyll content of wheat sprout is high. Wheat sprout further contains a wide variety of vitamins, minerals, and enzymes. Of the vitamins, especially vitamin A, vitamin K and vitamin E are present in very high concentrations. In addition to these, wheat sprout contains vitamin C, choline, thiamin, riboflavin, pyridoxine, vitamin B12, niacin, pantothenic acid, biotin and folic acid. As to the mineral content, it may be mentioned that wheat sprout contains especially great amounts of calcium, phosphorus and potassium, whereas its sodium content is low. In addition, wheat sprout contains significant amounts of copper, zinc and selenium, which have recently been proved to be excellent antioxidants.

Due to its composition, wheat sprout has been regarded as an excellent foodstuff, and it has in fact been used widely as so-called living food. Wheat sprout fibre is very coarse, and the human digestive tract is not able to digest it. For this reason, wheat sprout has not been used as such but in the form of juice.

Wheat sprout juice is usually prepared by extracting fresh wheat sprout e.g. in a juicer. The juice has to be used immediately after extraction, as it degenerates very rapidly. Besides moulding, the juice also oxidizes, thus losing its good properties. Wheat sprout juice also has a disagreeable smell. The rapid degeneration has prevented the development of satisfactory commercial products. Wheat sprout juice has been offered for sale in the form of ice cubes, but this has not provided a satisfactory solution to the storage and smell problems.

British Patent Specification 1 358 052 describes a process for producing an edible powder from unripe cereal, such as barley and wheat. In this patent specification, an attempt is made to solve the stability problems by adjusting the pH of the juice to 6 - 9, and by spray drying or lyophilizing the juice. According to the patent specification, the pH adjustment can be performed by the use of e.g. alkali metal carbonate or bicarbonate, ammonium hydroxide, calcium hydroxide, calcium carbonate or calcium glutamate. Heat treatment, such as heating at 90 to 150°C, is suggested to prevent enzymatic activity. It is also suggested that chemical preservatives, such as benzoic acid, methylparahydroxybenzoate or sodium benzoate, should be added to the product. Heating and use of chemical preservatives, however, are not acceptable in connection with living food nor are they recommendable for natural products.

Wheat sprout has also been used in cosmetic products and natural products and in homeopathy. Chlorophyll is known to have a skin regenerating effect. External application of wheat sprouts to refresh and sooth the skin and to treat both acne and various ulcers has been described in the literature of the art. In an attempt to solve the stability problems, wheat sprout or a preparation containing it has been e.g. sterilized or an anti-microbial agent has been added to the preparation.

EP Patent Application 279 984, for instance, describes pharmaceutical and cosmetic preparations containing an extract obtained from plants of the grass family and especially from cereal plants in combination with a pharmaceutically acceptable carrier or excipient. The carrier or excipient used in the preparations is an aqueous substance, and an anti-microbial agent is added to the preparations and possibly also ascorbic acid or β-carotene. Moreover, it is suggested that the extract should be sterilized before mixing with the carrier.

FR Patent Specification 1 039 340 describes the extraction of wheat germs by alcohol, and the use of the extract so obtained in hygiene products. Alcohol acts herein both as a solvent and as a preservative. However, the patent specification does not describe the stability properties of the extract.

It has now been found unexpectedly that the stability of wheat sprout juice can be significantly improved by adding thyme oil to the juice or a product containing it. Thyme oil is a known substance, which, due to its properties to be described below, is used widely e.g. in various pharmaceutical preparations, natural products and foodstuff products. Thyme oil is thus an acceptable and useful substance both physiologically and with regard to natural products.

Thyme is a very common plant having many species and varieties. The most common is *Thymus vulgaris L*., which contains about 0.8 to 2.6% of volatile oils, the quality and amount of which may vary greatly. Thyme contains e.g. phenols, terpene derivatives, such as para-cymene and γ-terpinene, alcohols, such as linalool, α-terpineol and 4-thujanol. Thymol is usually the major phenolic component, while carvacrol occurs in smaller amounts. In addition to these, thyme contains e.g. tannin, flavonoids, caffeic acid, ursolic acid and oleanolic acid.

Thyme has been used widely as folk medicine and in cosmetic substances and drugs. At all times it has been regarded as a very important medicinal plant, and it has been used, both fresh and dried, e.g. as an antithelmitic, antispasmodic, carminative, sedative, diaphoretic and coughing medicine, mainly in the form of a tincture or infusion. Thyme has also been used in the treatment of chronic gastritis and diarrhea and as an appetizer. Also, it has been used externally e.g. in health baths to relieve rheumatic symptoms and skin problems.

Modern medicine still uses thyme for the same purposes as above. Thyme oil is used as a flavour component, antispasmodic, carminative, appetizer, and expectorant in coughing medicines, in antiseptic mouth washes and liniments. Thymol is used similarly. In addition, it is used in the treatment of fungal skin infections and in dental formulations. Thyme oil has also been used in cosmetic products, such as toothpastes, soaps, detergents, creams, tonics, and perfumes.

Thyme has also been used both in cooking and in food industries. It is used therein as a spice either alone or in spice blends in bakery products, meat products, processed vegetables, relishes, gravies, and oils. Thyme oil in turn is used widely as a flavour and aroma component in almost all foodstuffs, including alcoholic and nonalcoholic beverages, dairy products, and meat products.

The use of thyme oil as a preserving agent e.g. in cosmetic products and drugs has riot been described in the literature. However, it has now been found unexpectedly that thyme oil improves significantly the stability of sprout juice and a preparation containing sprout juice. Thyme oil, also covers the disagreeable smell of sprout juice. Moreover, it has been found that thyme oil adds to the effects of sprout juice.

Accordingly, the invention relates to the use of thyme oil for improving the stability of wheat sprout juice and a preparation containing wheat sprout juice.

The invention also relates to a composition containing wheat sprout juice and thyme oil and suitable for both cosmetic and pharmaceutical uses.

The invention further relates to a process for producing a composition containing wheat sprout juice and thyme oil.

The composition can be formulated as various cosmetic and pharmaceutical preparations by using carriers, vehicles, and excipients known in the art. One skilled in the art is able to select the basic ingredients and other components best suited for the purpose according to the desired technological and physiological properties of the preparation. The preparation may be e.g. an anhydrous or aqueous cream, lotion, cleansing milk, tonic, hair care product, such as a conditioner. If desired, other components, such as other active agents, vitamins, minerals, fat components, aroma components, can be included in the preparation. A preferred embodiment is a cream preparation containing sprout juice and thyme oil. A cream preparation containing sprout juice and thyme oil and vitamin A, zinc and possibly vitamin E is most preferable.

The composition of the preferred preparation may be e.g. as follows:

| | |
|---|---|
| Vitamin A palmitate | 1 to 2 g |
| Zinc sulphate | 20 to 60 g |
| α-tocopherol acetate | 5 to 15 g |
| Lyophilized sprout juice powder | 10 to 25 g |
| Thyme oil | 5 to 12 g |
| Cream base ad | 1000.00 g |

The above-described composition has excellent properties in view of both pharmaceutical and cosmetic uses. The good properties of sprout juice were described above. The thyme of the composition contains thymol that revives and rejuvenates tissue. Thyme also promotes the regeneration of skin, also affecting the removal of dead tissue. In addition, thyme has a bactericidal effect. Zinc has an astringent effect on skin, it is antiseptic and softens skin and protects various rashes and minor wounds. Zinc is also an excellent moisturizer, preventing skin from drying. Moreover, zinc prevents cell damages caused by pollutants, microbes, ionizing radiation and chemical load formed both within the body and coming from outside the body. Vitamin A helps to keep the skin and mucosa in good condition and promotes the healing of dry and scaling skin. On the cell level, vitamin A resists cell changes caused by the radiation from the sun, among other things, such as the development of melanoma, and prevents cell damages caused by acidifying pollutants from the air and chemical pollutants.

The composition thus nourishes and regenerates skin tissue and increases the elasticity of skin. It also moisturizes dry skin, alleviates skin irritation and protects the skin. The composition rapidly soothes various infection reactions on the skin, and it has proved to be effective even in the treatment of very difficult eczema cases. The compositions according to the invention can thus be used e.g. as a base cream, cosmetic skin care product, after-sun product for soothing the skin, in the treatment of sun burns, and as a pharmaceutical substance in the treatment of wounds, bruises, and rashes. The composition may also be used in the treatment of scalp diseases.

Sprout juice is produced by growing wheat in a conventional way. Wheat sprouts are gathered when they are about 10 to 18 cm in length, and juice is extracted from them e.g. in a juicer or mixer. As the juice degenerates very rapidly, it is advisable to extract and store it cold. In the compositions according to the invention, sprout juice can be used as such, as a concentrate or lyophilized. Due to the rapid degeneration of the juice, lyophilizing is preferable in many cases.

The preparation is made by mixing sprout juice and thyme oil with the carrier or excipient and other components possibly used. The ingredients of the composition and the formulation of the preparation can be selected by one skilled in the art according to the desired end product. The preparation may be e.g. a cream, lotion, cleansing milk, tonic, hair care product, such as a conditioner. In addition to sprout juice and thyme oil, the preparation may contain conventional carriers, vehicles, other active agents, vitamins, minerals, fat components, aroma components.

A cream preparation containing sprout juice and thyme oil is preferred. When the preparation is a topical cream, a conventional commercial base cream or a conventional base cream prepared of individual raw materials may be used as a carrier, i.e. cream base. The cream base may be aqueous or anhydrous and its composition may also vary in other ways. The cream base is significant e.g. for the release of active agent(s) from the cream base and their absorption and migration e.g. through the skin into the blood circulation. One skilled in the art is able to select a cream base suited for a desired purpose, taking into account e.g. whether the cream should provide a systemic effect or a local effect, whether the releasing rate of the active agents from the cream is a critical feature, and whether the cream should have an occlusive effect, whereby it also moisturizes or hydrates the skin.

Active agents are usually released more rapidly from aqueous preparations (cremes) than from creams containing only fat. Fatty cream bases in turn improve the absorption of the active agent through the skin. This is due to their occlusion effect, whereby the hydration of skin improves its permeability.

Another factor to be taken into account in the formulation is the solubility of the active agent(s) in the cream base used. Solubility determines the type of the cream to be formulated to a great extent: a creme (water-in-oil or oil-in-water), liquid cream, or suspension cream. Most drugs are poorly soluble in fat. From the viewpoint of the releasing rate of the drug, a suspension cream is to be preferred to a liquid cream. On the other hand, from the viewpoint of the ease of use of suspension cream, it is necessary that the solids are sufficiently finely divided in order that they would not be felt as too coarse particles when the cream is applied.

Various compositions can be used as cream bases for the preparations containing sprout juice and thyme oil. As far as an anhydrous cream base is concerned, the cream type may be a "pure" suspension cream. Cremes in turn contain both water and a fat phase and additionally possibly various vehicles, such as preservatives and emulsifiers. A lotion can be prepared e.g. by using sprout juice as the water phase in combination with a suitable carrier.

The effect of the compositions according to the invention has been analyzed both through empirical use by prescribing application of the cream preparation e.g. once or twice a day as a cosmetic cream and through strictly controlled clinical experiments on diseases and skin problems of different types. The observed cosmetic effects included the moisturizing effect of the composition that made the skin elastic and smooth. After the application of the composition, the skin did no longer feel dry and no scaling or itching of the skin occurred. In addition, the skin became even and healthy in colour. The compositions also proved to be excellent anti-wrinkle formulas.

Clinical experiments were conducted on patients by applying the composition locally twice a day. The patients were women and men of different ages suffering from diseases varying from different types of eczema, including psoriasis, allergic symptoms and lupus erythematosus, to different types of wounds, rheumatoid arthritis and shingles. In the experiments, the appearance, extent and colour of the eczema areas were monitored, and the symptoms of the patients, such as itching and irritation, were recorded. In most cases, the healing effect of the composition became apparent after a few applications (in 2 to 3 days). After application for two weeks the symptoms had disappeared almost completely in all cases, the eczema areas had decreased clearly or receded completely, the colour of the skin had changed, and the skin was thicker and more elastic. In addition to the easily observable healing of eczema and other skin symptoms, a further advantage was that itching ceased almost immediately. After the application ended, the patients' symptoms did not reappear until in a few weeks or even months. In difficult allergy cases, the eczema and other symptoms, however, reappeared when an allergic reaction developed. It was found that the compositions were effective in the treatment of all of the diseases examined. In particular, it was found that they alleviated and healed e.g. allergic symptoms, infantile eczema, atopic eczema, and slight burns and wounds, including difficult varicose ulcers.

A few cases will be described below.

| Patient | Disease | Effect of the composition of the invention |
|---|---|---|
| 23-year-old man | allergy, atopic eczema especially in lower legs and thighs | the eczema healed up almost completely in 2 weeks |
| 28-year old woman | LDE, lupus erythematosus: rubescent, burning, smarting and tumid eczema area in the face | the eczema became considerably lighter in colour already in 5 days; the preparation also removed tumefaction |
| 10-year-old boy | atopic eczema | the eczema receded in 2 to 3 days |
| 31-year-old woman | unidentified eczema in both hands, ulcers and watery eczema between fingers | the eczema became nearly symptomless in a few weeks |
| X | psoriasis | scaling decreased after the 1st week of application; after the 2nd week, very little scaling occurred and the size of patches started to decrease |

The effect of thyme oil on the stability, smell and appearance of a wheat sprout juice composition

A cold-cream-type cream containing varying amounts of thyme oil was prepared for the first test. The composition of the cream was as follows:

| | |
|---|---|
| Lanoline | 500 g |
| Almond oil | 50 g |
| Wheat sprout juice | 500 g |
| Thyme | x g |

The cream was prepared by first mixing the lanoline, almond oil and thyme oil and adding the sprout juice to the obtained mixture, whereby a cold-cream-type green cream was obtained. The cream was stored at room temperature in 10 g ollas with the covers closed for 2 or 4 weeks. After storage the smell and colour and mould growth of the cream were checked. The results are shown in Tables 1 and 2.

**Table 1**

| **Effect of thyme oil on the growth of moulds in a sprout juice cream** | |
|---|---|
| Amount of thyme in cream g/1,000 g | Mould growth (4 weeks) |
| 0 | +++ |
| 2 | +++ |
| 4 | + |
| 8 | - |
| 16 | - |
| 32 | - |

| | |
|---|---|
| +++ = plenty of colonies, surface covered up | |
| + = only a few colonies < 5/olla | |
| - = no mould colonies | |

**Table 2**

| **Effect of thyme oil on the smell and colour of the sprout juice cream** | |
|---|---|
| Amount of thyme in cream g/1,000 g | Cream smell and colour |
| 0 | disagreeable, yellow |
| 2 | disagreeable, yellow |
| 4 | disagreeable, yellow |
| 8 | covering, medicinal, green |
| 16 | strongly medicinal, green |
| 32 | strongly medicinal, green |

The results show clearly that thyme oil prevents the growth of moulds and covers the strong disagreeable smell of sprout juice. It was also found that thyme oil prevents the colour of the surface of the sprout juice cream from turning from green to yellow.

The test was repeated by using preparation 5 described in Example 1, which contained 15 g of wheat sprout juice and 8 g of thyme oil per 1,000 g of the preparation. A corresponding preparation with no thyme oil was used as a control. The results are shown in Tables 3 and 4.

**Table 3**

| **Effect of thyme oil on the growth of moulds in a sprout juice preparation.** | |
|---|---|
| Preparation | Growth of moulds in the preparation (4 weeks) |
| Preparation 5 | - |
| Control | +++ |

| | |
|---|---|
| +++ = plenty of colonies, surface covered up | |
| - = no mould colonies | |

**Table 4**

| **Effect of thyme oil on the smell and colour of a sprout juice preparation.** | |
|---|---|
| Preparation | Smell and colour of the preparation |
| Preparation 5 | covering, medicinal, green |
| Control | disagreeable, yellow |

In a long-term follow-up, no mould growth or colour changes were detected in preparation 5 after four months.

Finally, the microbiological purity of wheat sprout juice, lyophilized wheat sprout juice powder, and the preparations described in Examples 1 to 5 was tested.

The microbiological purity of the sprout juice preparations (preparations 1 to 11) was tested by determining the total microbe growth and mould growth. The tests were performed in the following way:

A sample (5 to 10 g) to be assayed, a required amount of sterilized phosphate buffer (pH 6.8 to 7.4), and about 5% of sterile Tween 40 were introduced into a sterile glass erlenmeyer. The used amounts of the substances were recorded for subsequent determination of a sample dilution coefficient. The sample was extracted at about 40°C for about one hour with repeated shaking.

A 1 g sample of the extracted sample was poured into a sterile petri dish, and about 10 to 15 g of 40°C sterile culture medium agar solution was poured upon the sample. The dish was closed and rotated on a table surface along an 8-shaped path to mix the sample evenly into the culture medium. In the total microbe count, Casoagar, Difco, was used as a culture medium; in the mould count, Sabourad 4% maltose agar, Difco, was used. The dish was allowed to solidify, whereafter it was incubated with the agar side upwards for 2 days at +35°C (total microbe count) or for 5 days at room temperature (mould count).

After incubation the colonies present on the dish were counted, and the number of colonies was multiplied by a sample dilution coefficient so as to convert the count unit to colonies per gram of sample. The results obtained are shown in Table 5.

**Table 5**

| **Total microbe colonies and mould colonies counted per one gram of sample** | | |
|---|---|---|
| | Total microbes | Moulds |
| Preparation 1 | 50 | 0 |
| Preparation 2 | 40 | 0 |
| Preparation 3 | 80 | 0 |
| Preparation 4 | 40 | 0 |
| Preparation 5 | 70 | 0 |
| Preparation 6 | 60 | 0 |
| Preparation 7 | 30 | 0 |
| Preparation 8 | 80 | 0 |
| Preparation 9 | 60 | 0 |
| Preparation 10 | 100 | 0 |
| Preparation 11 | 20 | 0 |

As regards preparation 11, it is to be noted that it contained only about half of the amount of juice used in the other preparations. The smaller amount of juice may have contributed to the microbiological purity.

The results show that microbe growth occurred in all samples. Instead, no mould colonies were detected in any one of the samples during the incubation time. The completely anhydrous preparation 7 proved to have the highest microbiological purity; it showed little or no growth in all of the dishes analyzed. According to SLS (Finnish Drug Standards), a maximum of 100 microbes per one gram of cream is allowable for creams that are not intended for open wounds or burns. No standards are given for moulds, but one mould colony can be considered to correspond to one microbe colony.

In addition to the microbiological purity of the preparations, the microbiological purity of sprout juice and lyophilized sprout juice powder was also tested. Both were tested by incubating them for three and a half days at +35°C in a total microbe count dish. Significant growth was observed in both dishes, part of which was clearly mould growth.

To examine the pharmaceutical stability of the preparations, the prepared compositions were stored in closed 50 ml glass ollas that were packed almost full in room conditions and at elevated temperature (1 week +37°C). In addition, the preparations packed in the glass ollas were subjected to a freezing-and-thawing treatment, whereby each composition was frozen and thawed three times in a week. At the end of the stability test the preparations subjected to the thermal treatment and the freezing-and-thawing treatment were compared with preparations that had been stored at room temperature all the time. The following pharmaceutic-technological features were checked: separation of phases, breaking of emulsion state, recrystallizetion or separation of solids, and the smell of the product.

In the testing it was found that added water or water phase separated after the freezing-and-thawing treatment only with composition 1. The cream of composition 4 turned slightly more granular as a result of the freezing-and-thawing treatment; storing the cream at an elevated temperature of +37°C gave the same result.

In this test, preparations 2, 5, 10 and 11 were regarded as superior to the others. As regards the smell, the preparations stored in different conditions did not differ significantly from each other.

The invention will be described more closely by means of the following examples. The examples are given to illustrate the invention; they are not intended to limit the invention.

### Example 1

Production process 1. Production of a creme by the use of a ready cream base

The composition of the sprout juice cream was as follows:

| | |
|---|---|
| Vitamin A palmitate | 0.825 g |
| Zinc sulphate | 22.0 g |
| α-tocopherol(acetate) | 5.0 g |
| (sodium selenate | 2.5 g) |
| Lyophilized sprout juice | 7.5 g |
| Thyme oil | 4.0 g |
| Cream base ad | 500.0 g |

Vitamin A palmitate is a powder to be stored cool, which can be mixed in water but is not water-soluble. Zinc sulphate was added in the form of a powder which dissolved in water in the ratio of 1:0.6. α-tocopherolacetate is an oily liquid insoluble in water. Sodium selenate was in the form of a large-crystal powder readily soluble in water. It was added to some of the compositions. However, as sodium selenate is a disadvantageous substance from the cosmetic point of view, compositions with no sodium selenate were also prepared. In the stability/storage tests no difference was observed between compositions containing sodium selenate and compositions with no sodium selenate. Sprout juice was a lyophilized, flake-like powder poorly soluble in water. Thyme oil is an oily liquid insoluble in water. Water was distilled and it was at room temperature.

The formulated cream bases used in the preparations are listed below (the "code number" of the preparation, the water content of the cream, and the manufacturer of the cream base are indicated in brackets):
Hydran (1, 40%, Orion)
Neribase cremor (2, 68%, Leiras)
Neribase ungt (3, 30%, Leiras)
Ambilan (4, 50%, Orion)
Novalan (5, 60%, Orion)
Locobase (6, 30%, Brocades-Pharma)

1. Zinc sulphate and optionally sodium selenate were dissolved in a separate container in 20 g of distilled, room-temperature water. 2. Vitamin A palmitate and sprout juice powder, refined if required, were sieved through a 700 µm sieve. If desired, sprout juice particles were refined to correspond to a particle size of no more than 300 µm. The sieved ingredients were mixed manually into a homogenous cream paste while using 50 g of cream base, whereafter about 150 g of cream base were added followed by further mixing in a Kenwood mixer at a high mixing speed for a few minutes. Thyme oil and alpha-tocopherol were added to the obtained mixture followed by mixing at a high speed for a few minutes. The water solution prepared as described under item 1 was added to the mixture followed by mixing at a high speed for a few minutes. Finally, the rest of the cream base (ad 500 g) was added followed by mixing at a high speed for a few minutes.

### Example 2

Production process 2. Production of a suspension cream by using a ready cream base

The composition of the sprout juice cream was the same as in Example 1.

Formulated cream bases used in the preparations are listed below (the "code number" of the preparation, the water content of the cream, and the manufacturer of the cream base are indicated in brackets):
Neribase fat cream (7, 0%, Leiras)
Celestobase (8, 0%, Schering-Plough)

Zinc sulphate, vitamin A palmitate and sprout juice powder, refined if required, and optionally sodium selenate were sieved through a 700 µm sieve. If desired, sprout juice particles were refined to correspond to a particle size of no more than 300 µm. The sieved ingredients were mixed manually into a homogenous cream paste by using about 50 g of the cream base, whereafter about 150 g of the cream base were added followed by mixing in a Kenwood mixer at a high mixing power for a few minutes. Thyme oil and alpha-tocopherol were added to the obtained mixture followed by mixing at a high speed for a few minutes. Finally, the rest (ad 500 g) of the cream base was added followed by mixing at a high speed for a few minutes.

### Example 3

Production process 3. Production of an aqueous cream simultaneously with a cream base by using a sprout juice solution

The composition of the sprout juice cream was the same as in Example 1. For the part of the base cream the cream was prepared from individual raw materials, whereby the solids contained in the composition were added already in connection with the preparation of the cream base. Sprout juice was in the form of 3% juice instead of dry powder, whereby the 3% juice solution was used as water phase in the preparation of the creme base.

Fat phase (80 g of white petrolatum, 80 g of stearic acid, 80 g of cetaceum, 420 g of peanut oil, and 0.2 g of propyl parahydroxybenzoate) was weighed into a separate container, and the sprout juice and other substances to be added to the water phase (20 g of glycerol, 0.3 g of triethanolamine, optionally sodium selenate and zinc sulphate) were weighed into their own container. The amounts are calculated for 1,000 g of the preparation, which also contains water (ad 1,000). Solids were melted into the fat phase in a water bath, whereby the phase temperature was adjusted to about 60 to 70°C. The solids contained in the water phase were dissolved/suspended in the water phase and its temperature was adjusted in the water bath so that it was equal to the temperature of the fat phase. The fat phase was transferred to the container of a cream mixer, and vitamin A palmitate and alpha-tocopherol were added. The mixer was started and the water phase was added followed by mixing for about 5 minutes. Finally, thyme oil was added followed by mixing for about 5 minutes, thus obtaining preparation 9.

To prevent the separation of the phases, their cooling rate can be controlled, if required, by the use of e.g. a heat jacket or heat bath around the mixing container, whereby the phase in the mixing container remains all the time at a desired temperature.

### Example 4

Production process 4. Production of an aqueous cream simultaneously with a cream base by using dried sprout juice powder

The composition of the sprout juice cream was the same as in Example 1. The cream was prepared starting, for the part of the base cream, from individual raw materials, whereby the solids contained in the composition were added already in connection with the preparation of the cream base. Dried sprout juice powder was used as sprout juice. It was added by suspending.

Fat phase (120 g of white petrolatum, 120 g of stearic acid, 80 g of cetaceum, 420 g of peanut oil, and 0.2 g of propyl parahydroxybenzoate) was weighed into a separate container, and distilled water and other substances to be added to the water phase (240 g of glycerol, 0.3 g of triethanolamine, optionally sodium selenate and zinc sulphate) were weighed into their own container. Solids were melted into the fat phase in a water bath, whereby the phase temperature was adjusted to about 60 to 70°C. The solids contained in the water phase were dissolved/suspended in the water phase, and its temperature was adjusted in the water bath so that it was equal to the temperature of the fat phase. The fat phase was transferred to the container of a cream mixer, and vitamin A palmitate and alpha-tocopherol were added. The mixer was started and the water phase was added followed by mixing for about 5 minutes. Dried sprout juice powder was then added followed by mixing for about 5 minutes; finally, thyme oil was added followed by mixing for about 5 minutes, thus obtaining preparation 10.

To prevent the separation of the phases, their cooling rate can be controlled, if required, by the use of e.g. a heat jacket or heat bath around the mixing container, whereby the phase in the mixing container remains all the time at a desired temperature.

### Example 5

Production process 5. Production of a lotion or cream by using a sprout juice solution and a ready cream base

In this case, Celestobase cream base was used, in which 3% sprout juice was added instead of sprout juice powder.

12.7 g of zinc sulphate and optionally 1.4 g of sodium selenate were dissolved in a separate container in 89 g of 3% sprout juice. Vitamin A palmitate was sieved through a 700 µm sieve. 202 g of the cream base were weighed into the mixing container, sieved vitamin A palmitate was added followed by mixing in a Kenwood mixer at a high mixing power for a few minutes. 3.3 g of thyme oil and α-tocopherol were added into the mixture followed by mixing at a high speed for a few minutes, whereafter the water solution prepared as described above was added followed by mixing at a high speed for a few minutes.

Cream preparation 11 thus obtained contained only about half of the juice amount used in the other preparations. Nevertheless the preparation was rather fluid. It is also possible to add the same amount of juice as in the other preparations, whereby a lotion-type preparation 11 is obtained.

## Claims

1. Composition suitable for cosmetic and pharmaceutical uses, **characterized** in that it contains wheat sprout juice and thyme oil.

2. Composition according to claim 1, **characterized** in that it also contains a carrier and optionally vitamin A, vitamin E, and/or zinc.

3. Composition according to claim 1 or 2, **characterized** in that it is formulated as a cream preparation.

4. Process for producing a stable composition suitable for cosmetic and pharmaceutical uses, **characterized** by adding thyme oil to wheat sprout juice or to a composition containing wheat sprout juice.

5. Process for stabilizing wheat sprout juice, **characterized** by adding thyme oil to wheat sprout juice or a composition containing it.

6. Use of thyme oil as an agent improving the stability of wheat sprout juice or a preparation containing it.

## Patentansprüche

1. Für kosmetische und pharmazeutische Verwendungen geeignete Zusammensetzung,
dadurch **gekennzeichnet**, daß
sie Weizenkeimsaft und Thymianöl enthält.

2. Zusammensetzung gemäß Anspruch 1,
dadurch **gekennzeichnet**, daß
sie ebenfalls einen Träger und gegebenenfalls Vitamin A, Vitamin E und/oder Zink enthält.

3. Zusammensetzung gemäß Anspruch 1 oder 2,
dadurch **gekennzeichnet**, daß
sie als Creme-Zubereitung formuliert ist.

4. Verfahren zur Herstellung einer für kosmetische und pharmazeutische Verwendungen geeigneten stabilen Zusammensetzung, **gekennzeichnet** durch Zugabe von Thymianöl zu Weizenkeimsaft oder zu einer Zusammensetzung, die Weizenkeimsaft enthält.

5. Verfahren zur Stabilisierung von Weizenkeimsaft, **gekennzeichnet** durch Zugabe von Thymianöl zu Weizenkeimsaft oder einer diesen enthaltenden Zusammensetzung.

6. Verwendung von Thymianöl als Mittel zur Verbesserung der Stabilität von Weizenkeimsaft oder einer diesen enthaltenden Zusammensetzung.

## Revendications

1. Composition appropriée à des utilisations cosmétiques et pharmaceutiques, caractérisée en ce qu'elle contient du jus de germe de blé et de l'essence de thym.

2. Composition selon la revendication 1, caractérisée en ce qu'elle contient aussi un véhicule et éventuellement de la vitamine A, de la vitamine E et/ou du zinc.

3. Composition selon la revendication 1 ou 2, caractérisée en ce qu'elle se présente sous forme d'une préparation pour crème.

4. Procédé pour produire une composition stable, appropriée à à des utilisations pharmaceutiques et cosmétiques, caractérisé en ce que l'on ajoute de l'essence de thym à du jus de germe de blé ou à une composition contenant du jus de germe de blé.

5. Procédé pour stabiliser le jus de germe de blé, caractérisé en ce que l'on ajoute de l'essence de thym à du jus de germe de blé ou à une composition le contenant.

6. Utilisation de l'essence de thym comme agent améliorant la stabilité du jus de germe de blé ou d'une préparation le contenant.
